# EUROPEAN PATENT SPECIFICATION

(11) **EP 0 839 124 B1**
(45) Date of publication and mention of the grant of the patent: **05.04.2000**
(21) Application number: 96926364.9
(22) Date of filing: 16.07.1996
(51) Int. Cl.: C07C 67/38, C07C 69/24

(54) **PROCESS FOR THE CONTINUOUS CARBONYLATION OF OLEFINS**
VERFAHREN ZUR KONTINUIERLICHEN CARBONYLIERUNG VON OLEFINEN
PROCEDE DE CARBONYLATION CONTINUE D'OLEFINES

(30) Priority: 17.07.1995 EP 95201967
(43) Date of publication of application: 06.05.1998
(73) Proprietor: SHELL INTERNATIONALE RESEARCH MAATSCHAPPIJ B.V., 2596 HR Den Haag (NL)
(72) Inventor: DRENT, Eit, NL-1031 CM Amsterdam (NL); HASSELAAR, Melis, NL-1031 CM Amsterdam (NL)
(86) International application number: EP9603170
(87) International publication number: WO9703943

(56) References cited:
- EP-A- 0 186 228
- EP-A- 0 495 547
- EP-A- 0 577 205
- US-A- 5 495 041

## Description

The invention relates to a process for the continuous carbonylation of olefins whereby an olefin is reacted with carbon monoxide and a nucleophilic compound. In particular, the invention relates to the continuous carbonylation of ethene with carbon monoxide and a C1-6 alcohol.

Carbonylation of olefins with nucleophilic compounds in the presence of Metal Carbonyls ("Reppe reactions") has been described extensively in the standard textbook "New Syntheses with Carbon Monoxide", edited by J. Falbe (Springer-Verlag 1980). Hydrocarboxylation (i.e., wherein the nucleophilic compound is water) is dealt with in Section 3.6.2.1; hydroesterification (i.e., wherein the nucleophilic compound is an alcohol) is dealt with in Section 3.6.2.2, whereas Section 3.6.2.3 provides further samples of carbonylations of olefins with nucleophilic compounds other than water or an alcohol. Hydroesterification is of particular interest, as hydroesterification products, such as methyl propionate and n-butyl propionate are suitable replacements for the common chemical solvents ethyl acetate and n-butyl acetate.

In EP-A-0,411,721 a process for the continuous preparation of hydroesterification products is demonstrated, which process consumes smaller amounts of catalyst components per tonne of product than that of EP-A-0,279,477, which document also provides a process for the continuous carbonylation of olefins. Thus, alkyl propionates are prepared by hydroesterification in a reaction vessel in the presence of a carbonylation catalyst system based on palladium acetate, triphenylphosphine and methanesulphonic acid. The alkyl propionates are removed ("stripped") from the reaction vessel in a stream of vapour. Ligand consumption during the preparation of n-butyl propionate was reported to be as low as 3.3 to 6.8 kg/tonne, depending on the length of the trial run. The final rate of production (from an initial 500 mole product per mole Pd per hour) was 100 mole/mole.hr.

A deficiency of the continuous process of EP-A-0,411,721 (and EP-A-0,279,477) is the high ratio of triaryl monophosphine vis-à-vis the metal, and the coproduction of phosphonium salts due to the presence of methanesulphonic acid, a strong acid. Moreover, active catalyst is lost when the contaminants are removed. As a result, purity of the hydroesterification product is adversely affected, as well as the economy of the process. Finally, to provide a viable route for commercialisation, besides a highly desirable further reduction of ligand consumption, also improvement in respect of the production rate and retention thereof remains welcome. Replacement of the strong acid in this catalyst system by a weak acid such as a carboxylic acid would reduce the formation of phosphonium salts, however, it would also reduce the activity of the catalyst system. Besides, the weak acid undergoes reaction with the nucleophilic compound more easily and irreversibly than does the strong acid, thus increasing the content of contaminants.

A catalyst system that excels in the carbonylation of olefins is that disclosed in EP-A-0,495,547; found by combining (a) a source of Group VIII metal cations, such as palladium acetate, (b) a source of an aliphatic diphosphine, and (c) a source of anions. As anion source preferably a protonic acid is selected. Preference is in particular given to sources of non- or weakly coordinating anions, by which is meant that little or no covalent interaction takes place between the cation and the anion. If the anions are derived from a weak acid, e.g., a carboxylic acid, the carboxylic acid is preferably sterically hindered by bulky substituents to minimise the tendency for coordination. This document, however, provides no teaching to overcome the drawbacks identified in the continuous process mentioned above.

Another excellent catalyst system is disclosed in WO 94/18154. It differs from the catalyst system mentioned above in that it is a non-acidic catalyst system. Use of this catalyst system did also not provide the wanted continuous process with high rate of production, low impurity make and low ligand consumption.

Surprisingly, a continuous process has been found, which produces fewer contaminants, reduces the ligand consumption and improves the production rate by proper selection of the catalyst system. Accordingly, the present invention provides a process for the continuous carbonylation of olefins whereby an olefin is reacted with carbon monoxide and a nucleophilic compound in the presence of a catalyst system based on:
(a) a source of a Group VIII metal cations,
(b) a source of an aliphatic diphosphine, and
(c) a carboxylic acid which is the hydrocarboxylation product of the olefin,
whereby during the process the carboxylic acid is either added continuously or intermittently or is prepared in-situ by continuous or intermittent addition of water.

The olefin preferably is an alkene having from 2 to 20, more preferably from 2 to 6 carbon atoms per molecule. Most preferably, the olefin is ethene, producing desirable propionic derivatives.

Suitable co-reactants in the process of the invention include nucleophilic compounds other than water having one or more active (mobile) hydrogen atoms. The process of the invention is of particular interest for the hydroesterification of olefins (i.e., reaction with an alcohol), although it may also be applied when the nucleophilic compound is an amine, thioalcohol or acid, etc. Examples of suitable nucleophilic compounds are mono- and dihydric alkanols, such as methanol, ethanol, isopropanol, n-butanol, amyl alcohol, hexanol-1, ethylene glycol, butanediols and amines, such as ethylamine and diethylamine. Alkanols with 1 to 6 carbon atoms per molecule and alkanediols with 2 to 6 carbon atoms per molecule are preferred. n-Butanol, methanol and 1,4-butanediol are especially preferred. The use of these compounds as co-reactants enables the production of valuable carbonylation products, such as methyl propionate, butyl propionate, 1,4-diacyloxy butanes and (from butene) methyl pentanoate. These compounds are of commercial interest and may be used as solvents and in flavouring compositions and perfumes.

The process of the present invention can also be used to prepare carboxylic acids by continuous hydrocarboxylation of the olefin with water as nucleophilic compound. In that case, the presence of an additional anion source, such as the strong or weak acids as advocated in the relevant art, is not required, nor is the additional dosing (continuously or intermittently) of the carboxylic acid that is the hydrocarboxylation product of the olefin (as it is in-situ prepared).

The preferred choice for component (a) of the catalyst system is a source of cations of nickel, palladium and/or platinum, more preferably palladium. A suitable source for palladium cations are its salts, metallic palladium and (zero-valent) palladium complexes. Suitable sources for nickel and platinum cations are nickel salts of carboxylic acids and platinum complexes, for instance with acetylacetone). A very suitable source is palladium(II) acetate. Other similarly suitable sources may be found in the documents mentioned above.

Suitable aliphatic diphosphines have been disclosed in EP-A-0,495,547 and WO 94/18154. Thus, diphosphines that may be used in the process of the present invention have the general formula I

R¹R²P-R-PR³R⁴ (I)

wherein each of R¹, R², R³ and R⁴ independently represents a substituted or non-substituted aliphatic or cycloaliphatic group, or R¹ together with R² and P, and/or R³ together with R⁴ and P represent a substituted or non-substituted bivalent cyclic group with at least 5 ring atoms, or R¹ together with R², and/or R³ together with R⁴ represent a substituted or non-substituted bivalent cyclic group with at least 5 ring atoms whereby the two free valencies of such a cyclic group are linked to a single phosphorus atom, and R represents a bivalent organic bridging group containing from 1 to 4 atoms in the bridge.

Preferably, the bridging group R represents an alkylene group containing from 1 to 4 carbons atoms in the bridge, but it may also comprise a carbon chain, interrupted by a hetero atom, such as an oxygen atom. Most preferably, R represents a 1,2-ethylene, a 1,2-propylene or 1,3-propylene group, more in particular a 1,2-ethylene group.

Preferred aliphatic groups have 1 to 10, more preferably 1 to 6 carbon atoms. Examples of suitable groups are methyl, tert-butyl, cyclohexyl, etc.

Examples of phospha-cycloaliphatic groups comprise 1-phosphacyclohexyl, 2,6-dimethyl-1-phosphacyclohexyl, etc.

The use of a diphosphine comprising a bivalent (substituted) cyclic group, represented by R¹ together with R², and/or R³ together with R⁴, has been found to be most suitable. In general the cyclic group comprises at least 5 ring atoms and preferably contains from 6 to 9 ring atoms. More preferably the cyclic group contains 8 ring atoms. Substituents, if any, are usually alkyl groups having from 1 to 4 carbon atoms. As a rule, all ring atoms are carbon atoms, but bivalent cyclic groups containing one or two hetero atoms in the ring, such as oxygen- or nitrogen, atoms are not precluded. Examples of suitable bivalent cyclic groups are 1,4-cyclohexylene, 1,4-cycloheptylene, 1,3-cycloheptylene, 1,2-cyclooctylene, 1,3-cyclooctylene, 1,4-cyclooctylene, 1,5-cyclooctylene, 2-methyl-1,5-cyclooctylene, 2,6-dimethyl-1,4-cyclooctylene and 2,6-dimethyl-1,5-cyclooctylene groups, 1,4-cyclooctylene, 1,5-cyclooctylene, and methyl (di)substituted derivatives thereof being preferred.

Thus, the most preferred diphosphines of formula (I) are symmetric or asymmetric 1,5- or 1,4- isomers of 1,2-bis(cyclooctylenephosphino)ethane, and mixtures thereof (or in IUPAC nomenclature: the symmetric or asymmetric [3,3,1] or [4,2,1] isomers of 1,2-P,P'(9-phosphabicyclononyl)ethane). For the preparation of the bidentate ligands, reference is made to known techniques, for example the method disclosed in GB-A-1,127,965.

Component (c) of the catalyst system is different for each olefin used. Thus, if the olefin is ethene or propene, component (c) is either propionic acid or butyric acid. The advantage is that any reaction of the component (c) with the nucleophilic compound results in the same product as obtained by the carbonylation of the olefin with that nucleophilic compound. For instance, in case of hydroesterification the product is the same as obtained by esterification of the alcohol with the acid.

From EP-A-0,055,875 the use of water or a carboxylic acid is known to promote hydroesterification processes that are run batch-wise. However, this document is silent as regards the problem of loss of catalyst activity in continuous carbonylation processes. Besides, the processes have been conducted in the presence of a less active catalyst system, being based on a different ligand. Similarly, from EP-A-0,495,548 highly active catalyst systems based on aliphatic diphosphines substituted with tertiary alkyl groups are known, which again are only illustrated in batch-type experiments. Moreover, each of the experiments is conducted in the presence of a strong acid, pointing away from the inventive concept of the present invention.

The process is preferably operated under a blanket of inert gas, e.g., molecular nitrogen. The best results, in respect of ligand consumption, were found when all reagents were properly degassed (de-oxygenised), and charged to the reactor vessel under a blanket of this inert gas.

The catalyst compositions most preferably used comprise i) a palladium salt, ii) a bidentate ligand of the formula set out above, wherein R¹R²P and R³R⁴P each represent a 1,4- and/or 1,5- isomer of a cyclooctylenephosphino group and R represents a 1,2-ethylene, a 1,2-propylene or 1,3-propylene group, and iii) a carboxylic acid that is, in case the olefin is ethene, propionic acid.

The ratio of moles of ligands used per mole of cation preferably ranges from 0.5 to 10, most preferably from 1 to 3.

The quantity of, for instance in-situ prepared, carboxylic acid used in the carbonylation reaction may vary within wide ranges. Preferably, the amount of acid is 1 to 1000 mole, more preferably 1 to 500 mole of acid per mole of cation. To maintain a constant activity of the catalyst in the continuous process, acid or water are supplied intermittently, or -more preferably-continuously to the reaction vessel to avoid depletion of catalyst component c) by being stripped from the reaction vessel. Addition of acid or water in an amount ranging from 10⁻⁶ to 10⁻¹ mole per mole of product was found to be adequate (i.e., when carried out without liquid recycle. Else the amount will be less).

Conveniently, the amount of catalyst system is relatively small. Preferred amounts are in the range of 10⁻⁷ to 10⁻¹ mole cation per mole of olefin, more preferably in the range of 10⁻⁶ to 10⁻² on the same basis.

In the process of the invention liquid carbonylation product and/or surplus of either olefin or nucleophilic compound may serve as solvent during the reaction. It is also possible to perform the reaction in the presence of an additional liquid diluent, in particular when the reactants are used in stoichiometric amounts. Suitable diluents are for example polar, aprotic compounds, such as ketones, ethers (e.g., tetrahydrofuran or the dimethylether of diethyleneglycol) or sulphones (e.g., sulpholane). Preferably, the liquid carbonylation product serves as solvent.

The carbonylation reaction is conveniently carried out at moderate temperatures, generally ranging from 30 to 200 °C, preferably ranging from 50 to 150 °C. Reaction pressures may be atmospheric or superatmospheric. In particular pressures ranging from 2 to 70 bar gauge are preferred. Higher pressures are not precluded, but usually do not provide advantages.

The continuous process is preferably carried out in a stripping reactor, although it may also be conducted as a continuous process as disclosed in EP-A-0,279,477. In the preferred embodiment, the obtained reaction mixture is stripped from the liquid phase in the reaction vessel by vapour. Any other volatile materials present in the liquid phase, for example the nucleophilic compound, will also be removed in the stream of vapour. The rate at which gas is passed through the reaction vessel should be sufficient to strip the reaction product from the liquid phase, and may be determined by simple experimentation.

The stripping gas used to form the stream of vapour may comprise olefin, carbon monoxide and/or one or more inert gases, for example nitrogen, carbon dioxide and noble gases such as argon. In addition, the stripping gas may contain relatively minor amounts of hydrogen gas (e.g., up to a partial pressure of 1 bar a when the total pressure is in the preferred range). Preferably the gas consists of olefin and carbon monoxide, i.e., the feed. The molar ratio of olefin to carbon monoxide is preferably in the range of from 9:1 to 1:9, more preferably from 2:1 to 1:2.

The stream of vapour leaving the reaction vessel may be cooled to afford a gas phase and a liquid phase. The gas phase may be conveniently returned to the reactor vessel. In case the carbonylation product is an ester, the simplified process flow scheme disclosed in EP-A-0,411,721 provides a convenient method for separating the ester.

Although the invention is best explained by the process of preparing n-butyl propionate from n-butanol and ethene, the skilled reader will realise that the present invention has a much wider applicability. The invention will be illustrated by the following examples.

### Example 1 and Comparative Example A

A continuous process for the preparation of n-butyl propionate (NBP) from ethene and n-butanol (NBA) was carried out in a system comprising a 250 ml autoclave equipped with a gas sparging stirrer, a degasser and a distillation unit. The autoclave was provided with an outlet and with inlets for carbon monoxide, ethene, fresh catalyst and fresh and recycled liquid. Liquids (e.g., ligand dissolved in NBA and propionic acid dissolved in NBA) were dosed into the reactor vessel using high pressure HPLC pumps.

A typical experiment was started by setting the reactor level control at approximately 180 ml and filling the reactor vessel with (nitrogen blanketed) NBA/NBP mixture (weight ratio of 30/70) and the catalyst components carboxylic acid, ligand (a mixture of symmetric and asymmetric 1,5- and 1,4- isomers of 1,2-bis(cyclooctylenephosphino)ethane) and palladium acetate (in the amounts indicated in Table 1). Then, the reactor vessel was heated to reaction temperature (120 °C) under CO gas-flow and the reaction pressure was kept constant by a back-pressure regulator (6.0 bar gauge). Ethene was supplied in an equimolar ratio with CO (gas flow of about 60 Nl/hr). NBP was removed from the reactor vessel by stripping with excess CO and ethene, whilst leaving the catalyst solution available and active. The liquid hold-up of the reactor vessel was controlled by a level detector in the reactor and kept constant by supplying the alcohol NBA.

All gases leaving the condenser and the degasser passed through a recording wet gas meter. The gas consumption rate was used as a measure of the catalytic activity. Accordingly, stripped NBP was condensed, collected and analysed, for instance by on-line GLC.

In Example 1, 0.21 and 1.04 weight % propionic acid (wt%, on the contents of the reactor vessel) were added at respectively 29 and 48 hours. At 53 hours, 0.35 wt% water was added, followed by continuous addition of water after 73 hours of 0.83 wt%/hour.

The Table below provides detailed information on the process conditions and the production rate/ligand consumption.

### Comparative Examples B and C

These experiments were carried out as described above. However, the reactor vessel was filled with an NBA/NBP mixture of 20/80 weight/weight ratio. Gas flow in Comparative Example B was reduced form an initial 30 Nl/hr (0 to 4 hours) via 20 Nl/hr (4 to 25 hours) to 12 Nl/hr to accommodate for a decrease in production rate. In Comparative Example C it was about 20 Nl/hr. The carboxylic acid used in Comparative Example B was 2,6-di(sec-butyloxy)benzoic acid, as is in line with EP-A-0,495,547. In Comparative Example C a non-acidic catalyst (using 200.3 ml/l trihexylamine (= 6.74 wt%) as initial charge, whereto 6.78 wt% trihexylamine was dosed at 20.5 hours, 1.63 wt% of tri(n-butyl)orthoformate was dosed at 23.5 hours and 0.81 wt% trimethylorthoformate was dosed at 44 hours) as in line with WO 94/18154 was used. Details of these experiments are also included in the Table.

### Examples 2 to 4

These experiments were carried out as described above, however, at a reaction temperature of 130 °C, and a reaction pressure of 3.5 bar gauge. The reactor vessel was filled with an NBA/NBP mixture of 15/85 weight/weight ratio. Gas flow in Examples 2 and 3 was about 80 Nl/hr, that in Example 4 was about 60 Nl/hr.
In Example 2 propionic acid was continuously added (average dosing rate of 18 kg/ton ester). In Example 3, water was continuously added until 97 hours (av. dosing rate of 0.64 w% in product), whereupon propionic acid was continuously added (av. dosing rate of 14 kg/ton ester). In Example 4 propionic acid was continuously added (av. dosing rate of 36 kg/ton ester). Details of these experiments are also included in the Table.

### Example 5

Example 2 was repeated, however using 1 methoxy-2-propanol ("METHYL PROXITOL" a trademark) as nucleophilic compound to illustrate the potential for using other nucleophilic compounds. The initial reactor composition comprised 2.55 mmole/l Pd(II)acetate, 3.0 mmole/l ligand and 2.5 wt% acid. The final reactor composition comprised 2.41 mmole/l Pd(II)acetate and 0.8 wt% acid. At 1 hour ("initial time"), the average production rate was 345 mole/mole.hr. After 27 hours, the average production rate had fallen to 20 mole/mole.hr. The ligand consumption was compensated by average dosing of 0.21 kg/ton of ligand.

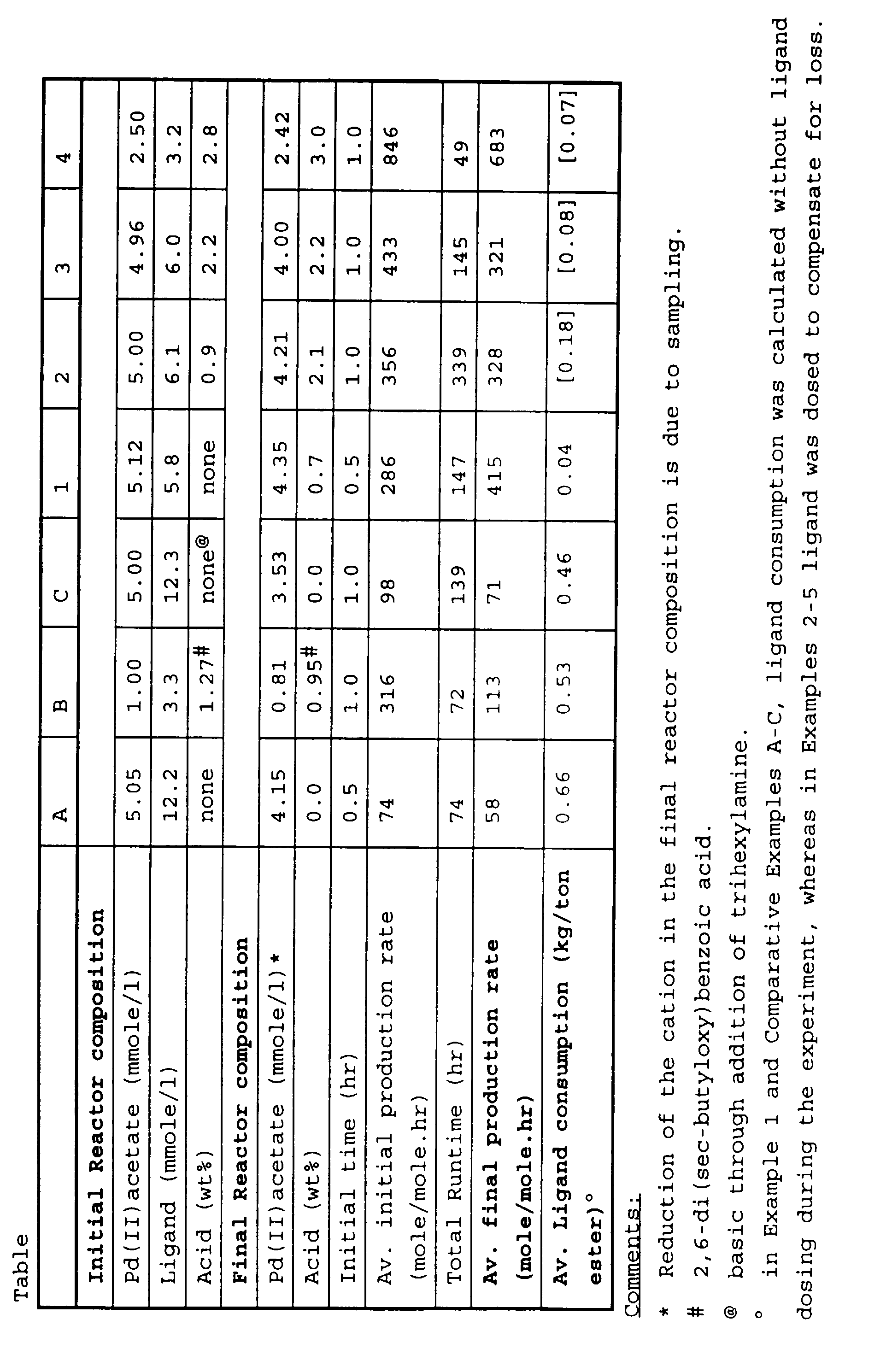

## Claims

1. A process for the continuous carbonylation of olefins whereby an olefin is reacted with carbon monoxide and a nucleophilic compound in the presence of a catalyst system based on:
(a) a source of a Group VIII metal cations,
(b) a source of an aliphatic diphosphine, and
(c) a carboxylic acid which is the hydrocarboxylation product of the olefin,
whereby during the process the carboxylic acid is either added continuously or intermittently or is prepared in-situ by continuous or intermittent addition of water.

2. A process as claimed in claim 1, wherein the olefin is ethene.

3. A process as claimed in claim 1 or 2, wherein the nucleophilic compound is an alkanol with 1 to 6 carbon atoms per molecule or an alkanediol with 2 to 6 carbon atoms per molecule.

4. A process as claimed in any one of claims 1 to 3, wherein component (a) of the catalyst system is a source of palladium cations.

5. A process as claimed in any one of claims 1 to 4, wherein component (b) of the catalyst system is a diphosphine having the general formula I
R¹R²P-R-PR³R⁴ (I)
wherein each of R¹, R², R³ and R⁴ independently represents a substituted or non-substituted aliphatic or cycloaliphatic group, or R¹ together with R² and P, and/or R³ together with R⁴ and P represent a substituted or non-substituted bivalent cyclic group with at least 5 ring atoms, or R¹ together with R², and/or R³ together with R⁴ represent a substituted or non-substituted bivalent cyclic group with at least 5 ring atoms whereby the two free valencies of such a cyclic group are linked to a single phosphorus atom, and R represents a bivalent organic bridging group containing from 1 to 4 atoms in the bridge.

6. A process as claimed in any one of claims 1 to 5, wherein component (b) of the catalyst system is a diphosphines of formula (I) being a symmetric or asymmetric 1,5- or 1,4- isomer of 1,2-bis(cyclooctylene-phosphino)ethane or a mixture.

7. A process as claimed in any one of claims 1 to 6, wherein the amount of catalyst system is in the range of 10⁻⁷ to 10⁻¹ mole cation per mole of olefin.

8. A process as claimed in any one of claims 1 to 7, wherein the liquid carbonylation product is used as carbonylation solvent.

9. A process as claimed in any one of claims 1 to 8, carried out in a stripping reactor.

10. A process as claimed in claim 9, wherein a stripping gas is used to form the stream of vapour, comprising olefin and carbon monoxide, i.e., the feed.

## Patentansprüche

1. Verfahren zur kontinuierlichen Carbonylierung von Olefinen, wobei ein Olefin mit Kohlenmonoxid und einer nucleophilen Verbindung in Gegenwart eines Katalysatorsystems umgesetzt wird, das auf
(a) einer Quelle von Kationen eines Gruppe VIII-Metalles,
(b) einer Quelle eines aliphatischen Diphosphins und
(c) einer Carbonsäure, die das Hydrocarboxylierungsprodukt des Olefins ist,
aufgebaut ist, wobei während des Verfahrens die Carbonsäure entweder kontinuierlich oder intermittierend zugesetzt wird oder in situ durch kontinuierliche oder intermittierende Zugabe von Wasser hergestellt wird.

2. Verfahren nach Anspruch 1, worin das Olefin Ethen ist.

3. Verfahren nach Anspruch 1 oder 2, worin die nucleophile Verbindung ein Alkanol mit 1 bis 6 Kohlenstoffatomen pro Molekül oder ein Alkandiol mit 2 bis 6 Kohlenstoffatomen pro Molekül ist.

4. Verfahren nach einem der Ansprüche 1 bis 3, worin die Komponente (a) des Katalysatorsystems eine Quelle von Palladiumkationen ist.

5. Verfahren nach einem der Ansprüche 1 bis 4, worin die Komponente (b) des Katalysatorsystems ein Diphosphin mit der allgemeinen Formel I
R¹R²P-R-PR³-R⁴ (I)
ist, worin jeder der Reste R¹, R², R³ und R⁴ unabhängig voneinander eine substituierte oder unsubstituierte aliphatische oder cycloaliphatische Gruppe darstellt oder R¹ zusammen mit R² und P und/oder R³ zusammen mit R⁴ und P eine substituierte oder unsubstituierte zweiwertige cyclische Gruppe mit wenigstens 5 Ringatomen bedeuten oder R¹ zusammen mit R² und/oder R³ zusammen mit R⁴ eine substituierte oder unsubstituierte zweiwertige cyclische Gruppe mit wenigstens 5 Ringatomen bedeuten, wobei die beiden freien Valenzen einer derartigen cyclischen Gruppe an ein einziges Phosphoratom gebunden sind, und worin R eine zweiwertige organische Brückengruppe mit 1 bis 4 Atomen in der Brücke darstellt.

6. Verfahren nach einem der Ansprüche 1 bis 5, worin die Komponente (b) des Katalysatorsystems ein Diphosphin der Formel (I) ist, wobei es sich um ein symmetrisches oder asymmetrisches 1,5- oder 1,4-Isomer von 1,2-Bis(cyclooctylenphosphino)ethan oder ein Gemisch hievon handelt.

7. Verfahren nach einem der Ansprüche 1 bis 6, worin die Menge des Katalysatorsystems im Bereich von 10⁻⁷ bis 10⁻¹ Mol Kation pro Mol Olefin liegt.

8. Verfahren nach einem der Ansprüche 1 bis 7, worin das flüssige Carbonylierungsprodukt als Carbonylierungslösungsmittel verwendet wird.

9. Verfahren nach einem der Ansprüche 1 bis 8, das in einem Strippreaktor ausgeführt wird.

10. Verfahren nach Anspruch 9, worin ein Strippgas zur Ausbildung des Dampfstroms verwendet wird, umfassend Olefin und Kohlenmonoxid, das heißt das Einsatzmaterial.

## Revendications

1. Procédé pour la carbonylation continue d'oléfines dans lequel on fait réagir une oléfine avec du monoxyde de carbone et un composé nucléophile en présence d'un système de catalyseur à base :
(a) d'une source de cations de métal du Groupe VIII,
(b) d'une source d'une diphosphine aliphatique, et
(c) d'un acide carboxylique qui est le produit d'hydrocarboxylation de l'oléfine,
dans lequel au cours du procédé l'acide carboxylique est ajouté de façon continue ou intermittente ou est préparé in situ par une addition continue ou intermittente d'eau.

2. Procédé suivant la revendication 1, dans lequel l'oléfine est de l'éthène.

3. Procédé suivant l'une ou l'autre des revendications 1 et 2, dans lequel le composé nucléophile est un alcanol avec 1 à 6 atomes de carbone par molécule ou un alcanediol avec 2 à 6 atomes de carbone par molécule.

4. Procédé suivant l'une quelconque des revendications 1 à 3, dans lequel le composant (a) du système de catalyseur est une source de cations de palladium.

5. Procédé suivant l'une quelconque des revendications 1 à 4, dans lequel le composant (b) du système de catalyseur est une diphosphine ayant la formule générale (I):
R¹R²P-R-PR³R⁴ (I)
dans laquelle chacun des R¹, R², R³ et R⁴ représente indépendamment un groupe aliphatique ou cycloaliphatique substitué ou non substitué, ou bien R¹ en même temps que R² et P, et/ou R³ en même temps que R⁴ et P représentent un groupe cyclique bivalent substitué ou non substitué avec au moins 5 atomes cycliques, ou bien R¹ en même temps que R², et/ou R³ en même temps que R⁴ représentent un groupe cyclique bivalent substitué ou non substitué avec au moins 5 atomes cycliques de sorte que les deux valences libres d'un tel groupe cyclique sont liées à un seul atome de phosphore, et R représente un groupe de pontage organique bivalent contenant de 1 à 4 atomes dans le pont.

6. Procédé suivant l'une quelconque des revendications 1 à 5, dans lequel le composant (b) du système de catalyseur est une diphosphine de formule (I) constituée d'un isomère 1,5 ou 1,4 symétrique ou asymétrique de 1,2-bis(cyclooctylènephosphino)éthane ou d'un mélange de ceux-ci.

7. Procédé suivant l'une quelconque des revendications 1 à 6, dans lequel la quantité de système de catalyseur se situe dans la gamme de 10⁻⁷ à 10⁻¹ mole de cation par mole d'oléfine.

8. Procédé suivant l'une quelconque des revendications 1 à 7, dans lequel le produit de carbonylation liquide est utilisé comme solvant de carbonylation.

9. Procédé suivant l'une quelconque des revendications 1 à 8, réalisé dans un réacteur d'extraction.

10. Procédé suivant la revendication 9, dans lequel on utilise un gaz d'extraction pour former le courant de vapeur, comprenant l'oléfine et le monoxyde de carbone, c'est-à-dire l'alimentation.
